# EUROPEAN PATENT APPLICATION

(11) **EP 2 628 436 A1**
(43) Date of publication of application: **21.08.2013**
(21) Application number: 12837651.4
(22) Date of filing: 15.10.2012
(51) Int. Cl.: A61B 1/00, A61B 1/12, G02B 23/24

(54) **ENDOSCOPE CAP MEMBER**

(30) Priority: 10.11.2011 JP 2011246740
(71) Applicant: OLYMPUS MEDICAL SYSTEMS CORP., Tokyo 151-0072 (JP)
(72) Inventor: ABE, Masanao, Tokyo 151-0072 (JP)
(74) Representative: Gunzelmann, Rainer
(86) International application number: PCT/JP2012/076586
(87) International publication number: WO 2013/069412

(57) **Abstract**

An endoscope plug member is an endoscope plug member that is installed in an opening portion of an endoscope having the opening portion which communicates with an outside, the endoscope plug member comprising: an opening portion side contact surface provided at the opening portion; a plug body having a plug body side contact surface that blocks the opening portion by contacting the opening portion side contact surface; attaching and holding means that holds the plug body movably in a direction in which the plug body side contact surface moves apart from the opening portion side contact surface, and is attachable to and detachable from the endoscope; a plug body urging member that gives an urging force that presses the plug body in a direction in which the plug body side contact surface contacts the opening portion side contact surface to the plug body; and a movable range adjusting portion that selectively changes a movable range of the plug body in the direction to move apart.

## Description

### Technical Field

The present invention relates to an endoscope plug member that is fitted to cylinder portions provided in an operation portion of an endoscope.

### Background Art

Endoscopes for use in a medical field are used for observation, inspection, treatment and the like by being inserted into bodies of patients. As endoscopes, various models are prepared, such as a nasal endoscope an insertion portion of which is inserted into a body from a nasal cavity, a peroral endoscope the insertion portion of which is inserted into a body from an oral cavity, and a colonoscope the insertion portion of which is inserted into a body from an anus.

In insides of the endoscopes, air/water feeding conduits, treatment instrument inserting conduits that also function as suction conduits and the like are provided. In order to avoid infection among patients, or infection from patients to surgeons, cleaning, disinfection or sterilization of outer surfaces and insides of respective conduits is immediately carried out in endoscopes after respective cases.

Japanese Patent Application Laid-Open Publication No. 8-308797 discloses the endoscope apparatus which makes cleaning operability favorable by enabling an operator to perform operation in the posture which does not place a burden on the operator, and reducing the number of injection operations by an injector as much as possible.

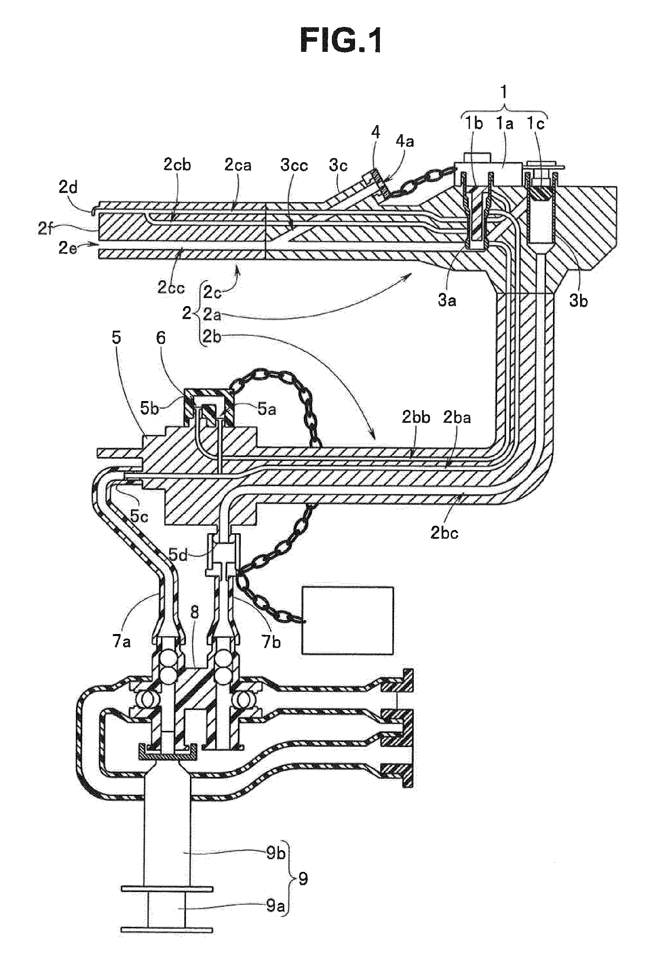

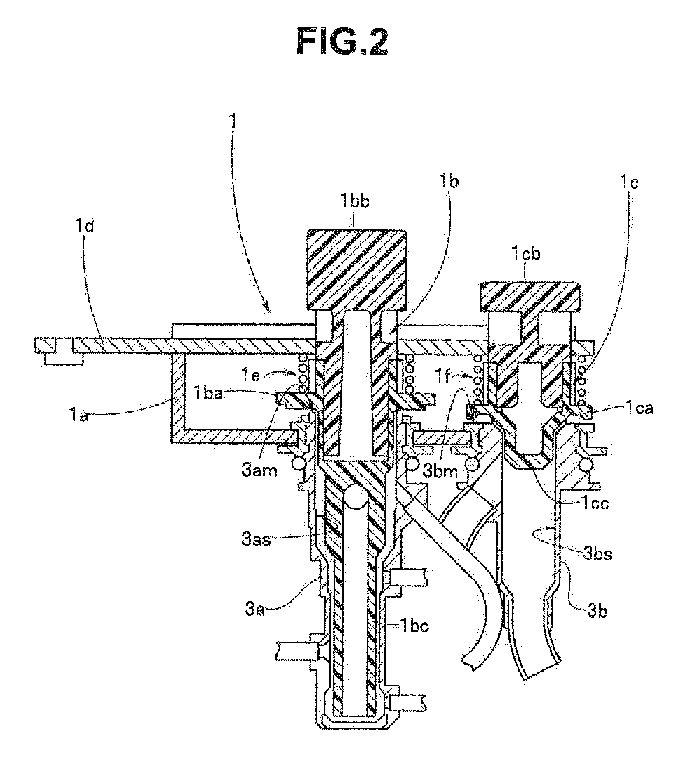

When an operator performs cleaning and disinfection of the conduits in the endoscope apparatus, the operator attaches an adapter main body 1a of an all conduits cleaning adapter (corresponding to the endoscope plug member of the present application) 1 to an operation portion 2a of an endoscope 2 as shown in Fig. 1 and Fig. 2. On the occasion, an air/water feeding plug 1b of the all conduits cleaning adapter 1 is loosely fitted in an air/water feeding cylinder 3a, and a suction plug 1c is loosely fitted in a suction cylinder 3b. Further, a plug body 4 having a leak hole 4a is installed in a forceps opening portion 3c that is provided at the operation portion 2a.

As shown in Fig. 1 and Fig. 2, the all conduits cleaning adapter 1 includes a holding plate 1d and springs 1e and 1f. The air/water feeding plug 1b is provided with a valve portion 1ba, a convex portion 1bb and a loose fitting portion 1bc. The suction plug 1c is provided with a valve portion 1ca, a convex portion 1cb and a loose fitting portion 1cc.

The loose fitting portion 1bc of the air/water feeding plug 1b is disposed in an internal space 3 as of the air/water feeding cylinder 3 a. The loose fitting portion 1cc of the suction plug 1c is disposed in an internal space 3bs of the suction cylinder 3b. One end surface side of the valve portion 1ba of the air/water feeding plug 1b is mounted on an opening end 3 am of the air/water feeding cylinder 3 a. One end surface side of the valve portion 1ca of the suction plug 1c is mounted on an opening end 3bm of the suction cylinder 3b. The convex portion 1bb of the air/water feeding plug 1b and the convex portion 1cb of the suction plug 1c are disposed on the holding plate 1d. The first spring 1e is disposed between the holding plate 1d and the other end surface side of the valve portion 1ba. The first spring 1e has an urging force that presses the valve portion 1ba to the opening end 3am of the air/water feeding cylinder 3 a with a pressure set in advance. The second spring 1f is disposed between the holding plate 1d and the other end surface side of the valve portion 1ca. The second spring 1f has an urging force that presses the valve portion 1ca to the opening end 3bm of the suction cylinder 3b with a pressure set in advance.

As shown in Fig. 1, a connecting plug 6 is installed onto a pressurizing pipe sleeve 5a and a water feeding pipe sleeve 5b of the connector portion 5 of the endoscope 2. Further, in the fitting state, one end portion of an air/water feeding conduit tube 7a is connected to an air feeding pipe sleeve 5c of the connector portion 5, and one end portion of a suction conduit tube 7b is connected to a suction pipe sleeve 5d. An injection tube body 8 is connected to the other end portion of the air/water feeding conduit tube 7a and the other end portion of the suction conduit tube 7b. An injector 9 of a capacity set in advance is connected to the injection tube body 8 at an air/water feeding conduit tube 7a side, for example.

Next, an operator operates a plunger 9a of the injector 9 and injects, for example, a liquid such as a cleaning liquid in a syringe 9b into the air/water feeding conduit tube 7a. The liquid is caused to flow into a cylinder proximal end side water feeding tube (hereinafter, abbreviated as a second water feeding tube) 2bb via an inside of a cylinder proximal end side air feeding tube (hereinafter, abbreviated as a second air feeding tube) 2ba that is inserted through an inside of a universal cord 2b, and the connecting plug 6.

With the operation of the plunger 9a, the liquid flows into the internal space 3 as of the air/water feeding cylinder 3 a from the second air feeding tube 2ba and the second water feeding tube 2bb. Here, part of the liquid that flows into the air/water feeding cylinder 3a pushes up the valve portion 1ba against the urging force of the first spring 1e, and is caused to flow out from the opening end 3am of the air/water feeding cylinder 3a. Thereby, the opening end 3am of the air/water feeding cylinder 3 a and an opening end side space are cleaned by the cleaning liquid.

Most of the liquid that flows into the air/water feeding cylinder 3a is caused to flow into a cylinder distal end side air feeding channel 2ca and a cylinder distal end side water feeding channel 2cb which are inserted through an inside of the insertion portion 2c of the endoscope 2 through the air/water feeding cylinder 3a. Thereafter, the liquid which flows into the channels 2ca and 2cb is caused to flow from an air/water feeding nozzle 2d that is provided at a distal end face 2f of the endoscope 2.

Thereby, an inside of the second air feeding tube 2ba, an inside of the second water feeding tube 2bb, an inside of the air/water feeding cylinder 3 a, an inside of the cylinder distal end side air feeding channel 2ca, and an inside of the cylinder distal end side water feeding channel 2cb are cleaned by the cleaning liquid.

Thereafter, the injector 9 is connected to a suction conduit tube 7b side of the injection tube body 8 and the plunger 9a is operated. Thereupon, the cleaning liquid passes through an inside of a cylinder proximal end side suction tube 2bc that is inserted through the inside of the universal cord 2b, an internal space 3bs of the suction cylinder 3b, and an inside of the cylinder distal end side suction channel 2cc which is inserted through an inside of the insertion portion 2c of the endoscope 2 from the suction conduit tube 7b, and is caused to flow out from the opening 2e, and cleaning by the cleaning liquid is performed.

Note that reference sign 3cc of Fig. 1 designates a forceps channel. The forceps channel 3cc has an opening in a forceps opening portion 3c, and communicates with the cylinder distal end side suction channel 2cc. Accordingly, part of the cleaning liquid which flows into the cylinder distal end side suction channel 2cc flows into the forceps channel 3 cc and is caused to flow out from the leak hole 4a. Thereby, the inside of the forceps channel 3cc is cleaned by the cleaning liquid.

Further, part of the liquid which flows into the suction cylinder 3b pushes up the valve portion 1ca against the urging force of the second spring 1f, and is caused to flow out from the opening end 3bm of the suction cylinder 3b. Thereby, the opening end 3bm of the suction cylinder 3b and the opening end side space are cleaned by the cleaning liquid.

The liquid that is injected into the respective conduits by the injector 9 is not limited to the cleaning liquid, but is sterilized water for rinsing, a disinfecting liquid or the like. Further, instead of injection of a liquid into the respective conduits by the injector 9, a gas such as air is fed, whereby drain in the conduits can be performed. Namely, injection of various fluids can be performed into the respective conduits by the injector 9.

However, in an attempt to fill and spread the liquid such as the cleaning liquid and the disinfecting liquid into an entire region of the conduits provided inside the endoscope, in particular into a side of the air/water feeding nozzle 2d located on the distal end side of the insertion portion 2c by liquid feeding means such as the injector 9, the liquid leaks from a gap between the valve portion 1ba and the opening end 3am, so that the number of times of operating the plunger of the injector, i.e. a time period (the number of times) of driving the liquid feeding means increases in comparison with a state where there is no such gap. Further, in an attempt to fill and spread the liquid into the entire region of the conduits, in particular into a side of the opening 2e located on the distal end side of the insertion portion 2c, the liquid leaks from a gap between the valve portion 1ca and the opening end 3bm, so that the time period (the number of times) of driving the liquid feeding means increases in comparison with the state where there is no such gap.

Further, in accordance with models, use purposes, configurations and the like of endoscopes, lengths, inside diameters and the like of conduits provided inside the endoscopes differ from one another. Further, in one endoscope, diameter dimensions differ in the conduit for air/water feeding and the conduit for suction.

For example, in the case where the fluid is caused to flow into the conduits of a nasal endoscope, and in the case where the fluid is caused to flow into the conduits of a colonoscope with a large conduit capacity relative to the nasal endoscope, the numbers of times of operating the plungers of the injectors differ. The number of times of operating the plunger of the injector increases when cleaning insides of conduits of the colonoscope having a large conduit volume in comparison with a case of cleaning insides of conduits of the nasal endoscope.

Further, in the case where the fluid is caused to flow into the air/water feeding conduit, and in the case where the fluid is caused to flow into the suction conduit with a large conduit capacity relative to the air/water feeding conduit, the numbers of times of operating the plunger of the injector differ, because the forceps channel is branched partway through the conduit in the suction conduit. The number of times of operating the plunger of the injector increases when cleaning an inside of the suction conduit in comparison with a case of cleaning an inside of the air/water feeding conduit.

Further, the all conduits cleaning adapter is configured to be attachable to all of a nasal endoscope, a peroral endoscope and a colonoscope which have different conduit capacities. In performing injection of the fluid by the injector in a state where the all conduits cleaning adapter is attached to the cylinder, in the nasal endoscope, part of the liquid that flows into the air/water feeding cylinder is caused to flow out from the opening end and most of the liquid flows into a cylinder distal end side air feeding channel and a cylinder distal end side water feeding channel through the air/water feeding cylinder. Thereafter, the liquid that flows into the air feeding channel and the water feeding channel on the cylinder distal end side is caused to flow from the air/water feeding nozzle, and thereby favorable cleaning can be performed.

However, in the colonoscope in which conduit lengths of the cylinder distal end side air feeding channel and the cylinder distal end side water feeding channel are large, and conduit volumes and fluid resistances inside conduits of the cylinder distal end side air feeding channel and the cylinder distal end side water feeding channel are larger than those of the nasal endoscope, the liquid flown into the air/water feeding cylinder flows out from the opening end more than necessary so that an amount of the fluid flown into the cylinder distal end side air feeding channel and the cylinder distal end side water feeding channel is decreased. As a result, the number of times of operating the plunger of the injector is increased, so that a burden on the operator is increased.

Further, in a case of supplying air into the conduit for the purpose of draining water, the air leaks from the opening end of the cylinder and thereby the number of times of operating the plunger of the injector is increased. Therefore, there have been demands from operators to spout air in a flash via the cylinder without any leakage from the opening end of the cylinder when supplying the air.

The present invention has been made in view of the foregoing circumstances, and an object of the present invention is to provide an endoscope plug member which is capable of performing an adjustment as to whether or not a fluid flown into an internal conduit of an endoscope is to be flown out from an opening portion of the endoscope, and in a case of flowing the fluid, capable of adjusting an amount of the flow, and which provides the fluid into the internal conduit in the optimal state to thereby enable cleaning, disinfection, rinsing and drain in the conduit.

### Disclosure of Invention

### Means for Solving the Problem

An endoscope plug member of one aspect of the present invention is an endoscope plug member that is installed in an opening portion of an endoscope having the opening portion which communicates with an outside, the endoscope plug member comprising: an opening portion side contact surface provided at the opening portion; a plug body having a plug body side contact surface that blocks the opening portion by contacting the opening portion side contact surface; attaching and holding means that holds the plug body movably in a direction in which the plug body side contact surface moves apart from the opening portion side contact surface, and is attachable to and detachable from the endoscope; a plug body urging member that gives an urging force that presses the plug body in a direction in which the plug body side contact surface contacts the opening portion side contact surface to the plug body; and a movable range adjusting portion that selectively changes a movable range of the plug body in the direction to move apart.

### Brief Description of the Drawings

Fig. 1 relates to a view explaining a schematic configuration of Japanese Patent Application Laid-Open Publication No. 8-308797, and is a view explaining a relation of an all conduits cleaning adapter and fluid conduits of an endoscope, and an injector;
Fig. 2 is a view explaining a relation of the all conduits cleaning adapter and cylinders;
Fig. 3 to Fig. 9 relate to one embodiment, and Fig. 3 is a view explaining fluid conduits of the endoscope and an endoscope plug member;
Fig. 4 is a sectional view explaining a configuration of the endoscope plug member;
Fig. 5 is a view of the endoscope plug member of Fig. 4 seen from an arrow Y5 direction;
Fig. 6 is a view of the endoscope plug member of Fig. 4 seen from an arrow Y6 direction, and is a view explaining a state in which a second contact surface is brought into close contact with a cylinder opening end;
Fig. 7 is a view of the endoscope plug member of Fig. 4 seen from the arrow Y6 direction, and is a view explaining a state in which a maximum open interval of the contact surface and the cylinder opening end is set at a first interval;
Fig. 8 is a view of the endoscope plug member of Fig. 4 seen from the arrow Y6 direction, and is a view explaining a state in which the maximum open interval of the contact surface and the cylinder opening end is set at a second interval wider than the first interval;
Fig. 9 is a view of the endoscope plug member of Fig. 4 seen from the arrow Y6 direction, and is a view explaining a state in which the maximum open interval of the contact surface and the cylinder opening end is set at a third interval wider than the second interval;
Fig. 10 is a view explaining an endoscope plug member in which the maximum open interval of the contact surface and the cylinder opening end is adjustable by a spacer being replaced;
Fig. 11 to Fig. 13 are modifications of a movable range adjusting portion, and Fig. 11 is a view explaining a movable range adjusting portion configured by including a through-hole in a plug body movement regulating member including an attaching and fixing rod and a knob mounting member;
Fig. 12 is a side view of the movable range adjusting portion shown in Fig. 11;
Fig. 13 is a view explaining a state in which the plug body movement regulating member is fixed to the knob mounting member;
Fig. 14 is a view explaining a movable range adjusting portion in which integral fixation of the plug body movement regulating member and the knob mounting member is performed by screwing of a male screw and a female screw; and
Fig. 15 is a view explaining a movable range adjusting portion in which pressurizing springs that respectively press a top surface of a knob portion of an air/water feeding conduit plug body and a top surface of a knob portion of a suction conduit plug body are provided at the plug body movement regulating member.

### Best Mode for Carrying Out the Invention

Hereinafter, an embodiment of the present invention will be described with reference to the drawings.

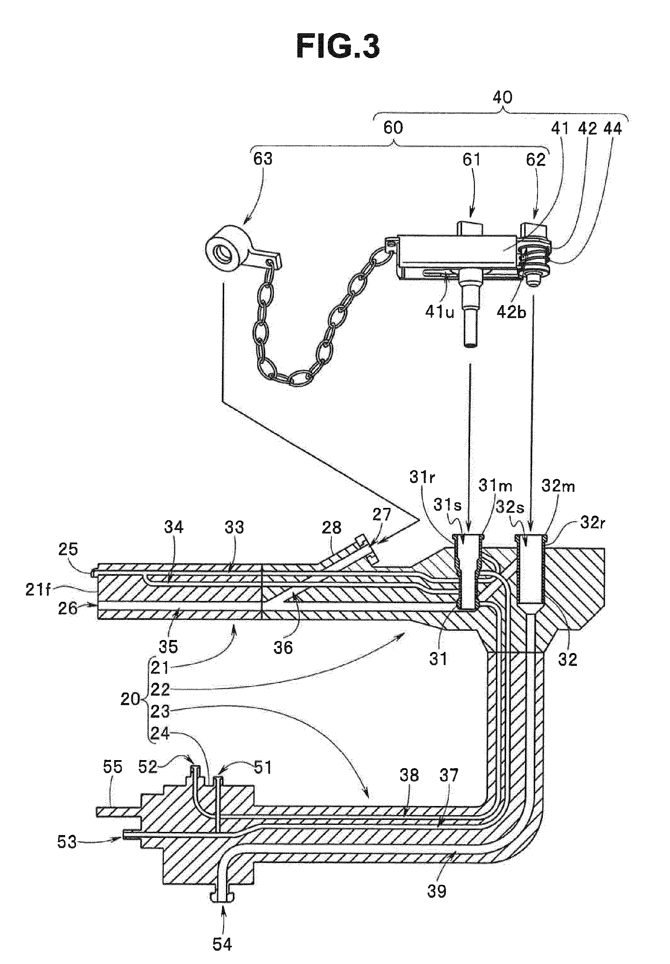

As shown in Fig. 3, an endoscope 20 is mainly configured by an insertion portion 21, an operation portion 22, a universal cord portion 23 and a connector portion 24. An air/water feeding nozzle 25 and a suction port 26 are provided on a distal end face of the insertion portion 21 in addition to an observation window and an illuminating window not illustrated. The air/water feeding nozzle 25 is an opening portion from which air/water feeding is performed toward the observation window in order to perform cleaning of the observation window. The suction port 26 is an opening portion for sucking tissue or the like of the inside of a body.

The connector portion 24 is provided with a pressurizing pipe sleeve 51, a water feeding pipe sleeve 52, a gas feeding pipe sleeve 53 and a suction pipe sleeve 54. Reference sign 55 designates a light guide connector. The pressurizing pipe sleeve 51 is an opening portion for feeding gas to a water feeding tank (not illustrated) to pressurize an inside of the water feeding tank. The water feeding pipe sleeve 52 is an opening portion to which a liquid is supplied from the water feeding tank. The gas feeding pipe sleeve 53 is an opening portion to which gas is supplied from a gas feeding apparatus (not illustrated). The suction pipe sleeve 54 is an opening portion to which a suction force is given by a suction apparatus (not illustrated).

In the present embodiment, the pressurizing pipe sleeve 51, the water feeding pipe sleeve 52, the gas feeding pipe sleeve 53 and the suction pipe sleeve 54 are injection ports for a fluid at a time of cleaning.

The operation portion 22 is provided with an air/water feeding cylinder 31 and a suction cylinder 32 which are opening portions having openings that communicate with an outside. Further, at a distal end side of the operation portion 22, a forceps opening portion 28 having a treatment instrument insertion port 27 which is an opening portion into which a treatment instrument is inserted.

Air/water feeding control button (not illustrated) is configured to be detachably installed into the air/water feeding cylinder 31. The air/water feeding control button is an operation button for performing an operation of selecting which is to be fed to the air/water feeding nozzle 25, a gas or a liquid.

A suction control button (not illustrated) is configured to be detachably installed into the suction cylinder 32. The suction control button is an operation button for performing an operation of selecting whether or not to perform suction from the suction port 26, and for housing sucked tissue or the like of the inside of the body.

When cleaning and disinfection of the air/water feeding conduit and the suction conduit which are fluid conduits are performed, an endoscope plug member 40 of the present invention is configured to be detachably installed into the opening portions provided at the operation portion 22 of the endoscope 20.

A distal end side air feeding channel 33 and a distal end side water feeding channel 34 are arranged at a distal end side of the operation portion 22 which is at a distal end side from the air/water feeding cylinder 31 of the endoscope 20 and at an interior of the insertion portion 21. The distal end side air feeding channel 33 and the distal end side water feeding channel 34 configure the air/water feeding conduit that is a fluid conduit and an internal conduit.

In the distal end side air feeding channel 33, a proximal end side communicates with the air/water feeding cylinder 31, and a distal end side communicates with the air/water feeding nozzle 25. In the distal end side water feeding channel 34, a proximal end side communicates with the air/water feeding cylinder 31, and a distal end side communicates with the air/water feeding nozzle 25 by merging (communicating) with the distal end side air feeding channel 33.

Further, a distal end side suction channel 35 that is a fluid conduit and an internal conduit, and configures a suction conduit is arranged at a distal end side of the operation portion 22 that is a distal end side from the suction cylinder 32 of the endoscope 20, and in the interior of the insertion portion 21. A proximal end side of the distal end side suction channel 35 communicates with the suction cylinder 32, and a distal end side thereof communicates with the suction port 26. Further, a branch portion is provided at an intermediate portion of the conduit of the distal end side suction channel 35. One end portion of a treatment instrument channel 36 communicates with the branch portion. The other end portion of the treatment instrument channel 36 communicates with the treatment instrument insertion port 27.

A proximal end side air feeding tube 37 and a proximal end side water feeding tube 38 are arranged at a proximal end side of the operation portion 22 that is a proximal end side from the air/water feeding cylinder 31 of the endoscope 20 and in interiors of the universal cord portion 23 and the connector portion 24. The proximal end side air feeding tube 37 and the proximal end side water feeding tube 38 configure an air/water feeding conduit that is a fluid conduit and an internal conduit. A distal end side of the proximal end side air feeding tube 37 communicates with the air/water feeding cylinder 31, and a proximal end side thereof is branched in an interior of a proximal end side from the air/water feeding cylinder 31 of the endoscope 20. One of the branched proximal en side air feeding tube 37 communicates with the pressurizing pipe sleeve 51, and the other one communicates with the gas feeding pipe sleeve 53. A distal end side of the proximal end side water feeding tube 38 communicates with the air/water feeding cylinder 31, and a proximal end side thereof communicates with the water feeding pipe sleeve 52.

Further, a proximal end side suction tube 39 that is a fluid conduit and an internal conduit, and configures the suction conduit is arranged at the proximal end side of the operation portion 22 that is at a proximal end side from the suction cylinder 32 of the endoscope 20, and in the interiors of the universal cord portion 23 and the connector portion 24. A distal end side of the proximal end side suction tube 39 communicates with the suction cylinder 32, and a proximal end side thereof communicates with the suction pipe sleeve 54.

The endoscope plug member 40 is configured by mainly including a plug body 60, an attaching and holding portion 41, a knob mounting member 42 that is attaching and holding means, and compressing springs 43 and 44. The plug body 60 is installed in the various opening portions provided in the endoscope 20 when the endoscope 20 is cleaned and disinfected. In the present embodiment, the plug body is installed in, for example, the opening portion of the treatment instrument insertion port 27, the opening portion of the air/water feeding cylinder 31 from which the air/water feeding control button (not illustrated) is detached, and the opening portion of the suction cylinder 32 from which the suction control button (not illustrated) is detached.

As shown in Fig. 3 to Fig. 5, the plug body 60 includes an air/water feeding conduit plug body 61, a suction conduit plug body 62 and a forceps opening portion plug body 63. The air/water feeding conduit plug body 61 is arranged in the air/water feeding cylinder 31. The suction conduit plug body 62 is arranged in the suction cylinder 32. The forceps opening portion plug body 63 is arranged in the forceps opening portion 28. The forceps opening portion plug body 63 has a leak hole (not illustrated) which is opened so that a preset quantity of fluid leaks.

The suction conduit plug body (hereinafter, abbreviated as the second plug body) 62 is made of a rigid resin such as polysulfone. The second plug body 62 is configured by mainly including a second knob portion 64, a second loose fitting portion 65 and a second valve portion 66. The second knob portion 64 configures one end portion of the second plug body 62, and includes a movable range adjusting portion that will be described later in the present embodiment.

The second loose fitting portion 65 is arranged in an internal space 32s of the suction cylinder 32 in a loosely fitted state. The second loose fitting portion 65 is disposed in the internal space 32s at an opening side of the suction cylinder 32. In the disposition state, a gap to be a fluid path is formed between an outer circumferential face of the second loose fitting portion 65 and an inner surface of the suction cylinder 32.

The second valve portion 66 is a flange portion provided on an outer circumferential face of the second plug body 62. The second valve portion 66 also functions as a compressing spring disposition portion. The second valve portion 66 includes a second contact surface 66t. The second contact surface 66t contacts a cylinder opening end 32m that is located at an operation portion external portion of the suction cylinder 32. An opposite surface from the second contact surface 66t is a second spring disposition surface 66b on which one surface of the second compressing spring 44 is disposed.

The air/water feeding conduit plug body (hereinafter, abbreviated as a first plug body) 61 is also made of a rigid resin such as polysulfone. The first plug body 61 is configured by mainly including a first knob portion 67, a first loose fitting portion 68 and a first valve portion 69. The first knob portion 67 configures one end portion of the first plug body 61, and includes a movable range adjusting portion similarly to the second plug body 62, in the present embodiment.

The first loose fitting portion 68 is arranged in an internal space 31s of the air/water feeding cylinder 31. The first loose fitting portion 68 is loosely disposed in the internal space 31s of the air/water feeding cylinder 31. In the disposition state, a gap to be a fluid path is formed between an outer circumferential face of the first loose fitting portion 68 and an inner surface that configures the internal space 31s.

The first valve portion 69 is a flange portion provided on an outer circumferential face of the first plug body 61. The first valve portion 69 also functions as a compressing spring disposition portion. The first valve portion 69 includes a first contact surface 69t. The first contact surface 69t contacts a cylinder opening end 31m that is located at an operation portion outer portion of the air/water feeding cylinder 31. A back surface of the first contact surface 69t is a first spring disposition surface 69b, on which one surface of the first compressing spring 43 is disposed.

Note that in the aforementioned embodiment, such a configuration that blocks the opening by causing the first contact surface 69t of the first plug body 61 to contact the cylinder opening end 31m of the air/water feeding cylinder 31 is adopted. Further, such a configuration that blocks the opening by causing the second contact surface 66t of the second plug body 62 to contact the cylinder opening end 32m of the suction cylinder 32 is adopted.

However, the contact surface of the cylinder 31 which the contact surface of the plug body 61 contacts may be provided on an inner surface or an outer circumferential face of the cylinder 32, instead of the opening end. Likewise, the contact surface of the cylinder 32 which the contact surface of the plug body 62 contacts may be provided on an inner surface or an outer circumferential face of the cylinder 32, instead of the opening end.

More specifically, a flange or a step portion that is protruded to a center axis side is provided at the inner surface of the air/water feeding cylinder 31, a surface at the opening side, of the step portion is set as the contact surface of the cylinder 31. Alternatively, the flange or the step portion that protrudes to a center axis side is provided at the inner surface of the suction cylinder 32, and a surface at the opening side, of the flange or the step portion is set as the contact surface of the cylinder 32.

Further, a flange or a step portion that protrudes to an outer side is provided at an outer circumferential face side of the air/water feeding cylinder 31 in such a manner as to surround the opening end, and a surface at an opening side of the flange or the step portion is set as the contact surface of the cylinder 31. Alternatively, a flange or a step portion that protrudes to the outer side is provided at the outer circumferential face side of the suction cylinder 32 in such a manner as to surround the opening end, and a surface at the opening side of the flange or the step portion is set as the contact surface of the cylinder 32.

A configuration that blocks the opening of the air/water feeding cylinder 31 by causing the contact surface 69t of the plug body 61 to contact the contact surface of the cylinder 31 may be adopted, and a configuration that blocks the opening of the suction cylinder 32 by causing the contact surface 66t of the plug body 62 to contact the contact surface of the cylinder 32 may be adopted.

The attaching and holding portion 41 and the knob mounting member 42 are configured by a metal plate member such as SUS, for example, having rigidity set in advance. The knob mounting member 42 is in an elongated flat shape. In the knob mounting member 42, a through-hole 42a in which the plug body 61 is mounted, and a through-hole 42b in which the plug body 62 is mounted are formed.

In contrast with this, the attaching and holding portion 41 is formed by the metallic plate member being bent. The attaching and holding portion 41 is configured by including an attaching surface 41 a, a pair of holding portions 41 b, and a gap forming portion 41c. The attaching surface 41a has a U-shaped slit 41u. The holding portion 41b is attaching and holding means. The holding portion 41b has an opposing surface 41f which opposes one surface of the attaching surface 41a. The gap forming portion 41c is provided at a side of one short side of the attaching surface 41a. The gap forming portion 41c is formed by a distal end face being bent toward the opposing surface 41f. The knob mounting member 42 is slidably disposed between the distal end face of the gap forming portion 41c and the opposing surfaces 41f of a pair of holding portions 41b.

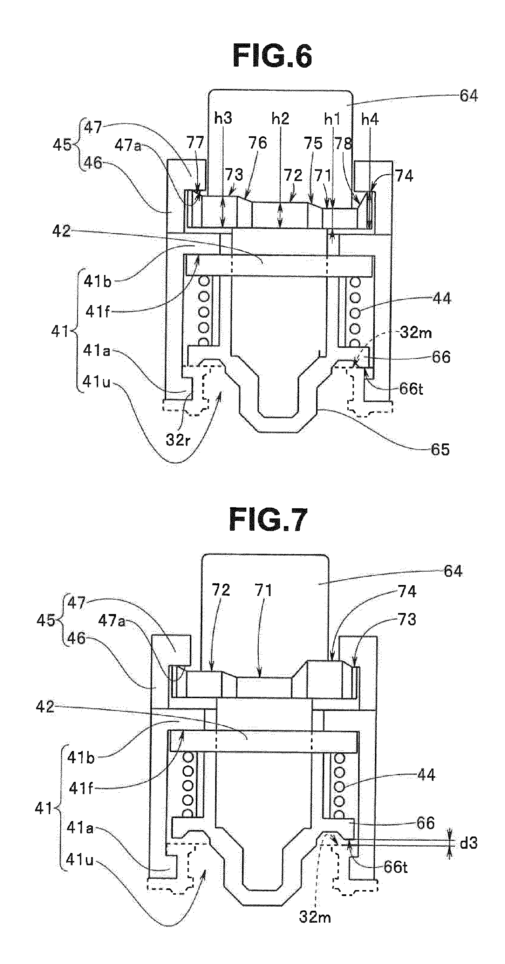

Note that an opening side of the U-shaped slit 41u is at the side of the other short side of the attaching surface 41a. As shown in Fig. 6, the attaching surface 41a which forms the U-shaped slit 41u is fitted in the air/water feeding cylinder groove 31r which is located at an opening portion outer side of the air/water feeding cylinder 31 of the endoscope 20 and the suction cylinder groove 32r at an opening portion outer side of the suction cylinder 32. As a result, the attaching and holding portion 41 is positioned and fixed to the endoscope 20. Note that the U-shaped slit 41u opens to configure a fluid drain port 41m.

An installing method of the endoscope plug member 40 to the various opening portions provided in the operation portion 22 of the endoscope 20 will be described.

To the opening portion of the treatment instrument insertion port 27, the forceps opening portion plug body 63 is installed by being fitted.

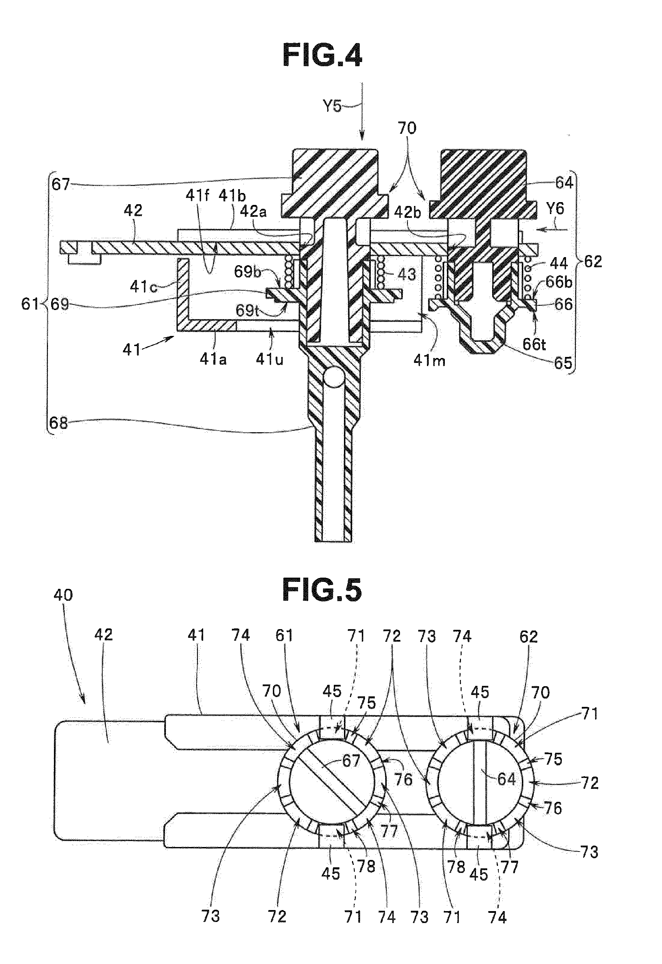

With respect to the opening portion of the air/water feeding cylinder 31, the first plug body 61 is installed by being fitted, and with respect to the opening portion of the suction cylinder 32, the second plug body 62 is installed by being fitted. On the occasion, the knob mounting member 42 is first brought into a state in which the knob mounting member 42 is slid to the side where the first plug body 61 and the second plug body 62 are mounted, that is, to the right side in Fig. 4, with respect to the attaching and holding portion 41. Subsequently, in this state, the first loose fitting portion 68 of the first plug body 61 is inserted into the air/water feeding cylinder 31, and the second loose fitting portion 65 of the second plug body 62 is inserted into the suction cylinder 32.

Thereafter, the attaching and holding portion 41 is slid to the side where the first plug body 61 and the second plug body 62 are mounted, that is, the right side in Fig. 4 with respect to the knob mounting member 42. On the occasion, the attaching surface 41a which forms the U-shaped slit 41u is fitted in the air/water feeding cylinder groove 31r and the suction cylinder groove 32r. As a result, the attaching and holding portion 41 is positioned and fixed to the endoscope 20.

Namely, the attaching and holding portion 41 and the knob mounting member 42 are attaching and holding means that holds the first plug body 61 and the second plug body 62 movably in the opening directions of the air/water feeding cylinder 31 and the suction cylinder 32, and can be installed on the endoscope 20.

In a state in which the attaching and holding portion 41 is positioned and fixed to the endoscope 20, the air/water feeding conduit compressing spring (hereinafter, abbreviated as the first compressing spring) 43 is an urging member that has an urging force that presses the first contact surface 69t of the first valve portion 69 of the first plug body 61 to the cylinder opening end 31 m of the air/water feeding cylinder 31. The first compressing spring 43 is disposed between the knob mounting member 42 and the first spring disposition surface 69b of the first valve portion 69.

In a state in which the attaching and holding portion 41 is positioned and fixed to the endoscope 20, the suction conduit compressing spring (hereinafter, abbreviated as the second compressing spring) 44 is an urging member that has an urging force that presses the second contact surface 66t of the second valve portion 66 of the second plug body 62 to the cylinder opening end 32m of the suction cylinder 32. The second compressing spring 44 is disposed between the knob mounting member 42 and the second spring disposition surface 66b of the second valve portion 66.

As shown in Fig. 5, Fig. 6 and the like, the knob portions 64 and 67 of the present embodiment are respectively provided with knob portion flanges 70 that are cam portions to be movable range adjusting portions. The holding portion 41b of the attaching and holding portion 41 is provided with a pair of knob position defining portions 45 to be a movable range adjusting portion.

Note that in the following description, configurations of the knob portion flanges 70 are the same in the first knob portion 67 and the second knob portion 64. Therefore, the configuration about the knob portion flange 70 of the second knob portion 64 will be described, whereas the description of the knob portion flange 70 of the first knob portion 67 will be omitted.

The knob portion flange 70 is a flange portion provided at an outer circumference of the second knob portion 64. A plurality of cam surfaces are formed on a front surface side of the knob portion flange 70. A back surface of the knob portion flange 70 is a plane, and contacts a surface at an opposite side from the opposing surfaces 41f in a pair of holding portions 41b of the attaching and holding portion 41. By the back surface contacting the surface, the movable position (range) of the second plug body 62 to the second loose fitting portion 65 side by the urging force of the second compressing spring 44 is regulated.

A plurality of cam surfaces of the knob portion flange 70 are configured by including, for example, a first plane 71, a second plane 72, a third plane 73, a fourth plane 74, and inclined surfaces 75, 76, 77 and 78.

The first plane 71 is set at a height dimension h1 that is set in advance from a cam bottom surface that is the back surface of the knob portion flange 70. The second plane 72 is set at a height dimension h2 that is set in advance from the cam bottom surface. The third plane 73 is set at a height dimension h3 that is set in advance from the cam bottom surface. The fourth plane 74 is set at a height dimension h4 that is set in advance from the cam bottom surface.

The first inclined surface 75 is provided between the first plane 71 and the second plane 72. The second inclined surface 76 is provided between the second plane 72 and the third plane 73. The third inclined surface 77 is provided between the third plane 73 and the fourth plane 74. The fourth inclined surface 78 is provided between the fourth plane 74 and the first plane 71. A plurality of cam surfaces form a continuous surface on the front surface of the knob portion flange 70.

The knob position defining portion 45 is configured by including a defining portion main body 46 and a contact portion 47. A bottom surface of the defining portion main body 46 is integrally fixed to a pair of respective holding portions 41b of the attaching and holding portion 41 by bonding, welding or the like. Note that in the present embodiment, the knob position defining portion 45 is fixed to the attaching and holding portion 41 by bonding, welding or the like. However, for example, a part of the attaching and holding portion 41 may be extended from the holding portion 41b, and may form the knob position defining portion.

The contact portion 47 protrudes from the defining portion main body 46. The contact portion 47 includes a defining surface 47a. Any one of a plurality of cam surfaces of the knob portion flange 70 contacts the defining surface 47a. The cam surface contacts the defining surface 47a, thereby regulates a movable position (range) of the first plug body 61 to the first knob portion 67 side, and regulates the movable position (range) of the second plug body 62 to the second knob portion 64 side. Namely, in the state in which the attaching and holding portion 41 is positioned and fixed to the endoscope 20, the cam surface contacts the defining surface 47a, defines an interval between the cylinder opening end 31m of the air/water feeding cylinder 31 and the first contact surface 69t of the first plug body 61, and defines an interval between the cylinder opening end 32m of the suction cylinder 32 and the second contact surface 66t of the second plug body 62.

Here, a cleaning method of the endoscope 20 in the present embodiment will be shown.

First, by the method mentioned above, the endoscope plug member 40 is installed into the various opening portions provided in the operation portion 22 of the endoscope 20.

A connecting plug 6 illustrated in Fig. 1 is installed in the pressurizing pipe sleeve 51 and the water feeding pipe sleeve 52 of the connector portion 24 of the endoscope 20. Further, in the state, one end portion of the air/water feeding conduit tube 7a illustrated in Fig. 1 is connected to the gas feeding pipe sleeve 53 of the connector portion 24, and one end portion of the suction conduit tube 7b is connected to the suction pipe sleeve 54. An injection tube body 8 illustrated in Fig. 1 is connected to the other end portion of the air/water feeding conduit tube 7a and the other end portion of the suction conduit tube 7b. Subsequently, an injector 9 of a capacity set in advance illustrated in Fig. 1 is connected to, for example, the air/water feeding conduit tube 7a side of the injection tube body 8.

Next, the operator operates a plunger 9a of the injector 9 to inject a liquid such as a cleaning liquid, for example, into a syringe 9b to the air/water feeding conduit tube 7a. Thereupon, the liquid is caused to flow into the proximal end side water feeding tube 38 via the inside of the proximal end side air feeding tube 37 which is inserted through the inside of the universal cord portion 23 and the connecting plug 6.

With the operation of the plunger 9a, the liquid flows into the internal space 31s of the air/water feeding cylinder 31 from the proximal end side air feeding tube 37 and the proximal end side water feeding tube 38. Here, part of the liquid which flows into the air/water feeding cylinder 31 pushes up the first plug body 61 against the urging force of the first compressing spring 43. Thereupon, part of the liquid is caused to flow out from the gap between the first valve portion 69 and the cylinder opening end 31m. As a result, cleaning or the like by the liquid for the internal space 31s of the air/water feeding cylinder 31 and the cylinder opening end 3 1 m is performed.

Most of the liquid which flows into the air/water feeding cylinder 31 flows into the distal end side air feeding channel 33 and the distal end side water feeding channel 34 which are inserted through the inside of the insertion portion 21 of the endoscope 20 through the air/water feeding cylinder 31. Thereafter, the liquid which flows into the channels 33 and 34 are caused to flow out from the air/water feeding nozzle 25 which is provided at the distal end face 2f of the endoscope 20. The air/water feeding nozzle 25 is a discharge port of the liquid at the time of cleaning.

By the above, cleaning or the like by the liquid for the inside of the proximal end side air feeding tube 37, the proximal end side water feeding tube 38, the inside of the air/water feeding cylinder 31, the inside of the distal end side air feeding channel 33 and the inside of the distal end side water feeding channel 34 is performed.

Thereafter, the injector 9 is connected to the suction conduit tube 7b side of the injection tube body 8 and operates the plunger 9a. Thereupon, the liquid passes through the inside of the distal end side suction tube 39 which is inserted through the inside of the universal cord portion 23, the internal space 32s of the suction cylinder 32, the inside of the distal end side suction channel 35 and the inside of the treatment instrument channel 36, from the suction conduit tube 7b. A part of the liquid which passes is caused to flow out from the leak hole of the forceps opening portion plug body 63, a part thereof is caused to flow out from the gap between the second valve portion 66 which is pushed up and the cylinder opening end 32m, and most thereof is caused to flow out from the suction port 26, whereby cleaning or the like by the liquid is performed. The suction port 26 becomes a discharge port of the liquid at the time of cleaning.

At this time, in the present embodiment, the attaching and holding portion 41 is positioned and fixed to the endoscope 20 as shown in Fig. 6, the second knob portion 64 is rotated in this state, and the fourth plane 74 of the cam surface of the knob portion flange 70 is disposed at the position where the fourth plane 74 contacts the defining surface 47a of the knob position defining portion 45, whereby the second contact surface 66t of the second valve portion 66 of the second plug body 62 and the cylinder opening end 32m of the suction cylinder 32 are in close contact with each other. Note that in the first plug body 61, the first knob portion 67 is also rotated, and the fourth plane 74 is also disposed at the position where the fourth plane 74 contacts the defining surface 47a, whereby the first contact surface 69t of the first plug body 61 and the cylinder opening end 31m of the air/water feeding cylinder 31 are also in close contact with each other.

The second knob portion 64 is operated and the third plane 73 of the cam surface of the knob portion flange 70 is disposed on the defining surface 47a of the knob position defining portion 45 as shown in Fig. 7. By this, a maximum open interval between the second contact surface 66t of the second plug body 62 and the cylinder opening end 32m of the suction cylinder 32 is set at d3. Note that the first knob portion 67 is operated and the third plane 73 is disposed on the defining surface 47a, whereby a maximum open interval between the first contact surface 69t of the first plug body 61 and the cylinder opening end 31m of the air/water feeding cylinder 31 is set at d3. Namely, d3 is the distance which satisfies d3=h4-h3.

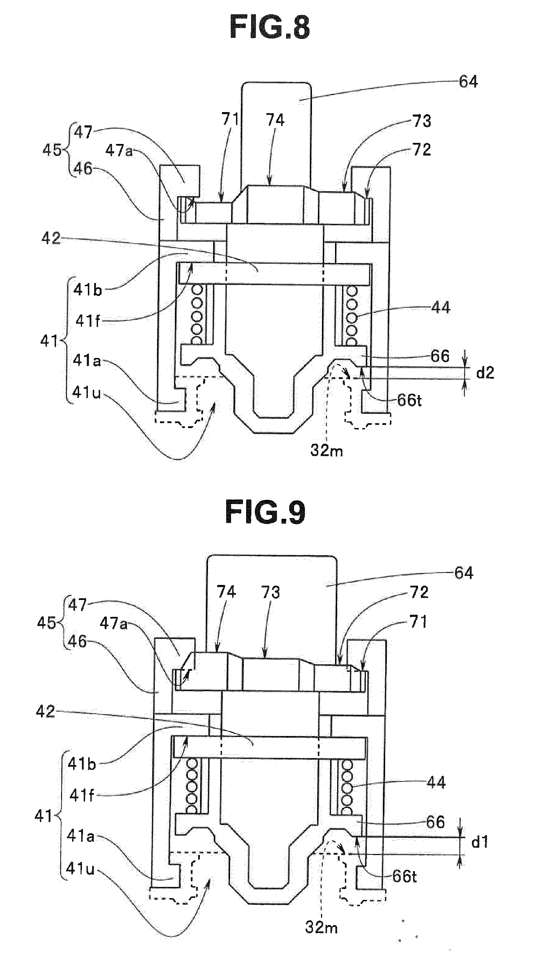

Likewise, the second plane 72 of the cam surface of the knob portion flange 70 is disposed on the defining surface 47a of the knob position defining portion 45 as shown in Fig. 8, whereby the maximum open interval between the second contact surface 66t of the second plug body 62 and the cylinder opening end 32m of the suction cylinder 32 is set at d2. Note that with respect to the first knob portion 67, the second plane 72 is disposed on the defining surface 47a, whereby the maximum open interval between the first contact surface 69t of the first plug body 61 and the cylinder opening end 31m of the air/water feeding cylinder 31 is set at d2. Namely, d2 is the distance which satisfies d2=h4-h2.

Further, the first plane 71 of the cam surface of the knob portion flange 70 is disposed on the defining surface 47a of the knob position defining portion 45 as shown in Fig. 9, whereby the maximum open interval between the second contact surface 66t of the second plug body 62 and the cylinder opening end 32m of the suction cylinder 32 is set at d1. Note that with respect to the first knob portion 67, the first plane 71 is disposed on the defining surface 47a, whereby the maximum open interval between the first contact surface 69t of the first plug body 61 and the cylinder opening end 31m of the air/water feeding cylinder 31 is set at d1. Namely, d1 becomes the distance which satisfies d1=h4-h1, and d1>d2>d3.

As above, in the present embodiment, any one of the first plane 71 to the fourth plane 74 of the cam surface of the knob portion flange 70 is disposed on the defining surface 47a of the knob position defining portion 45, whereby the first contact surface 69t and the cylinder opening end 31m can be brought into a close contact state, the second contact surface 66t and the cylinder opening end 32m can be brought into a close contact state, or the maximum open interval can be defined to be the dimensions d1 , d2 and d3 which are set in advance.

The intervals d1, d2 and d3 are defined to correspond to the conduit capacity, whereby the gap between the contact surface 66t of the second valve portion 66 and the cylinder opening end 32m, and the outflow amount of the liquid such as a cleaning liquid that flows out from the gap can be adjusted to be optimal, and the outflow amount of the liquid such as the cleaning liquid that flows out from the gap between the contact surface 69t of the first valve portion 69 and the cylinder opening end 31m can be adjusted to be optimal.

Further, the first contact surface 69t and the cylinder opening end 31m are brought into a close contact state, whereby the liquid and air can be quickly supplied to all regions of the air/water feeding conduit provided in the interior of the endoscope 20, in particular, to the side of the air/water feeding nozzle 25 which is located at the distal end side of the insertion portion 21 without the liquid and air leaking out from the cylinder opening of the air/water feeding cylinder 31. As a result, filling of the liquid into the air/water feeding conduit, or discharge of moisture or the like in the air/water feeding conduit by air can be performed quickly.

Further, the second contact surface 66t and the cylinder opening end 32m are brought into a close contact state, whereby the liquid and air can be quickly supplied to all regions of the suction conduit provided in the interior of the endoscope 20, in particular, to the side of the suction port 26 which is located at the distal end side of the insertion portion 21, without the liquid and air leaking out from the cylinder opening of the suction cylinder 32. As a result, filling of the liquid into the suction conduit, or discharge of moisture or the like in the suction conduit by air can be performed quickly.

Further, in the endoscope with a large conduit capacity such as a colonoscope, for example, the maximum open interval is set at d3. Thereby, when a liquid such as a cleaning liquid is injected into the air/water feeding conduit, an optimal amount of cleaning liquid or the like is caused to flow out from the cylinder opening of the air/water feeding cylinder 31. Further, when a liquid such as a cleaning liquid is injected into the suction conduit, an optimal amount of the cleaning liquid or the like is caused to flow out from the cylinder opening of the suction cylinder 32. In other words, while a cleaning liquid or the like more than necessary is prevented from flowing out from the cylinder opening, cleaning of the cylinder opening end 31m, or cleaning of the cylinder opening end 32m can be performed. Accordingly, cleaning or the like can be performed by the cleaning liquid or the like being supplied to the distal end side air feeding channel 33 and the distal end side water feeding channel 34 through the air/water feeding cylinder 31, and cleaning or the like can be performed by the cleaning liquid or the like being supplied to the distal end side suction channel 35 and the treatment instrument channel 36 through the suction cylinder 32, without the number of operation times of operating the plunger 9a of the injector 9 being increased.

Further, in an endoscope such as a peroral endoscope, for example, with the conduit capacity smaller than a colonoscope and larger than a nasal endoscope, the maximum open interval is set at d2. In a nasal endoscope or the like, the maximum open interval is set at d1. As a result, when a fluid such as a cleaning liquid is injected into the air/water feeding conduits of the respective endoscopes, optimal amounts of the cleaning liquid or the like are caused to flow out from the cylinder openings of the air/water feeding cylinders 31. Further, when a fluid such as a cleaning liquid is injected into the suction conduits of the respective endoscopes, optimal amounts of the cleaning liquid or the like are caused to flow out from the cylinder openings of the suction cylinders 32. In other words, the cleaning liquid or the like more than necessary is prevented from flowing out from the cylinder openings, and cleaning of the cylinder opening ends 31m or cleaning of the cylinder opening ends 32m can be performed. Accordingly, cleaning or the like can be performed by the cleaning liquid or the like being supplied to the distal end side air feeding channels 33 and the distal end side water feeding channels 34 via the air/water feeding cylinders 31 of the respective endoscopes, and cleaning or the like can be performed by the cleaning liquid or the like being supplied to the distal end side suction channels 35 and the treatment instrument channels 36 via the suction cylinders 32 of the respective endoscopes, without the number of operation times of operating the plunger 9a of the injector 9 being increased.

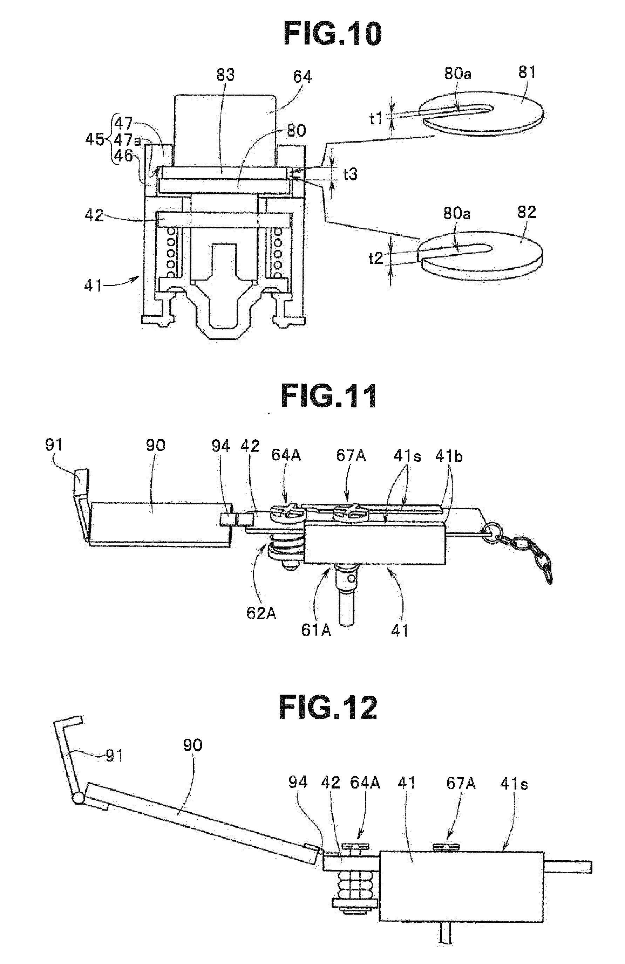

Note that in the embodiment described above, the cam surfaces including, for example, the planes 71 to 74 are provided on the knob portion flanges 70 provided at the knob portions 64 and 67. However, as shown in Fig. 10, a knob portion flange 80 corresponding to, for example, the interval d1 is provided at each of the knob portions 64 and 67, a first spacer 81 of a thickness t1, a second spacer 82 of a thickness t2, or a third spacer 83 of a thickness t3 is disposed on the knob portion flange 80, whereby the operation and the effect described above may be obtained. Reference sign 80a designates a knob release groove. In the present embodiment, the knob portion flange 80 and the spacers 81 to 83 function as the movable range adjusting portion.

More specifically, in the state in which the spacer is not mounted on the knob portion flange 80, the operation and the effect shown in Fig. 9 are obtained. In contrast with this, the first spacer 81 is mounted on the knob portion flange 80, whereby the operation and the effect shown in Fig. 8 are obtained. Further, the second spacer 82 is mounted on the knob portion flange 80, whereby the operation and the effect shown in Fig. 7 are obtained. The third spacer 83 is mounted on the knob portion flange 80, whereby the operation and the effect shown in Fig. 6 are obtained.

Note that the spacers 81, 82 and 83 described above may be respectively provided at the knob portions 64 and 67, or may be integrally provided. Namely, the spacers 81, 82 and 83 may be formed into the shape in which the spacers 81, 82 and 83 can be integrally loaded on the knob portion flanges of the knob portions 64 and 67.

Fig. 11 to Fig. 13 are modifications of the movable range adjusting portion.

As shown in Fig. 11 and Fig. 12, in the present embodiment, the movable range adjusting portion is a plug body movement regulating member 90.

As shown in Fig. 11 and Fig. 12, a plug body 61 A of the present embodiment includes a knob portion 64A, and a plug body 62A includes a knob portion 67A. Top surfaces of the knob portions 64A and 67A are configured by respectively having planes. The plane configured on the top surface of the knob portion 64A and the plane configured on the top surface of the knob portion 67A are configured to protrude by a preset amount with respect to upper side surfaces 41s of a pair of holding portions 41b.

The plug body movement regulating member 90 is configured by a metallic plate member such as SUS having rigidity set in advance. One end of the plug body movement regulating member 90 is rotatably provided at one end of the knob mounting member 42 by a hinge member 94. A hook member 91 that is engaged with the other end of the knob mounting member 42 is rotatably provided at the other end of the plug body movement regulating member 90.

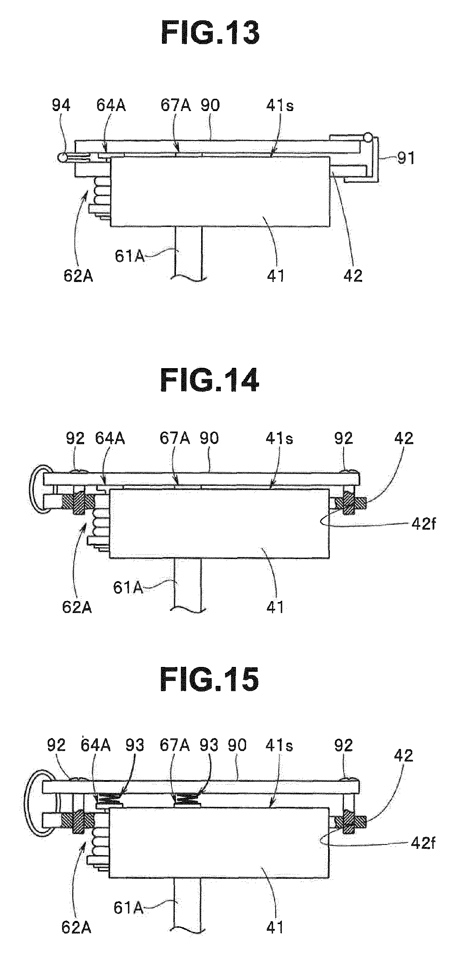

In the present embodiment, the hook member 91 is fixedly provided by being engaged with the other end of the knob mounting member 42 as shown in Fig. 13, whereby one surface of the knob mounting member 42 is disposed in contact with the planes configured on the top surfaces of the knob portions 64A and 67A. At this time, similarly to the aforementioned Fig. 6, for example, the second contact surface 66t of the second valve portion 66 and the cylinder opening end 32m of the suction cylinder 32 are brought into a close contact state, and the first contact surface 69t of the first valve portion 69 and the cylinder opening end 31m of the air/water feeding cylinder 31 are brought into a close contact state.

In the present embodiment, the hook member 91 of the plug body movement regulating member 90 is engaged with the other end of the knob mounting member 42, and the plug body movement regulating member 90 and the knob mounting member 42 are brought into an integrally fixed and provided state which is set in advance. Thereupon, a state can be brought about in which filling of a liquid, or discharge of moisture and the like in the conduits can be performed quickly without the liquid and air leaking out from the cylinder openings of the respective cylinders 31 and 32, for example.

The plug body movement regulating member 90 is combined with the embodiment described above, and the number of planes provided on the cam surface of the knob portion flange 70 may be decreased, or the number of spacers may be decreased. Note that in the case of the combining configuration, the planes of the aforementioned configuration are formed on the top surfaces of the knob portions 64 and 67.

Further, integral fixation of the plug body movement regulating member 90 and the knob mounting member 42 may be performed by screwing as shown in Fig. 14. More specifically, male screws 92 are provided at both side portions of the plug body movement regulating member 90, female screws 42f are provided at both side portions of the knob mounting member 42, whereby the respective screws are screwed on each other and both the end portions are integrally fixed.

According to the configuration, both the end portions of the plug body movement regulating member 90 are integrally fixed to the knob mounting member 42 by screwing, whereby the knob portions 64A and 67A can be uniformly compressed.

Note that in this case, one end of the plug body movement regulating member 90 and one end of the knob mounting member 42 are not limited to fixation by the hinge member 94. Namely, as shown in Fig. 14, openings are provided in one end of the plug body movement regulating member 90 and one end of the knob mounting member 42, and an annular member is inserted through the openings, whereby the plug body movement regulating member 90 may be movably fixed to the knob mounting member 42.

Further, positioning of the other end side of the plug body movement regulating member 90 and the other end side of the knob mounting member 42 may be performed by an attaching and fixing rod, and a fixing through-hole 42h. The attaching and fixing rod is provided to protrude from one surface opposing the knob mounting member 42 in the other end side of the plug body movement regulating member 90. The fixing through-hole 42h is provided at the other end side of the knob mounting member 42. The attaching and fixing rod is disposed to penetrate through the fixing through-hole 42h. At this time, the plug body movement regulating member 90 is positioned in the direction orthogonal to the movable direction of the plug bodies 61A and 62A, with respect to the knob mounting member 42, and becomes movable in the movable direction of the plug bodies 61A and 62A.

In this case, by the amount by which the plug body movement regulating member 90 is pushed into the plug bodies 61A and 62A side, the amounts of the liquid and air leaking out from the cylinder openings of the respective cylinders 31 and 32 can be adjusted.

Furthermore, as shown in Fig. 15, pressurizing portions 93 may be provided at the plug body movement regulating member 90 to oppose the knob portions 64A and 67A. The pressurizing portion 93 is an urging member formed by a spring member in a plate shape being bent, or an urging member formed by a coil-shaped spring member, or an elastic member such as sponge.

According to the configuration, the pressurizing portion 93 presses the top surfaces of the knob portions 64A and 67A. As a result, an opening/closing degree of the first contact surface 69t of the first valve portion 69 and the cylinder opening end 31m of the air/water feeding cylinder 31, and an opening/closing degree of the second contact surface 66t of the second valve portion 66 and the cylinder opening end 32m of the suction cylinder 32 by fluid pressure change.

Namely, when a supply pressure which is given to the first plug body 61 by the cleaning liquid which is supplied into the air/water feeding cylinder 31 through the gas feeding pipe sleeve 53 is a predetermined pressure, the movable range of the plug body 61A changes in a case in which the pressurizing spring 93 presses the top surface of the knob portion 64A and in a case in which the pressurizing spring 93 does not press the top surface of the knob portion 64A. Further, when a supply pressure which is given to the second plug body 62 by the cleaning liquid which is supplied into the suction cylinder 32 through the suction pipe sleeve 54 is a predetermined pressure, the movable range of the plug body 62A changes in a case in which the pressurizing spring 93 presses the top surface of the knob portion 67A and in a case in which the pressurizing spring 93 does not press the top surface of the knob portion 67A.

Accordingly, an urging force of the pressurizing spring 93 is properly set, and thereby the endoscope plug member corresponding to the conduit capacity of an endoscope can be obtained. In the configuration, the male screws 92 are screwed into a predetermined state to the female screws 42f, whereby the contact surface 66t of the valve portion 66 and the cylinder opening end 32m of the cylinder 32 can be brought into a close contact state, and the contact surface 69t of the valve portion 69 and the cylinder opening end 31m of the cylinder 31 can be brought into a close contact state.

Note that the present invention is not limited to only the embodiment described above, and various modifications can be carried out within the range without departing from the gist of the invention.

For example, the plug body described in the aforementioned embodiment of the present application, for example, the plug body having the configuration of the knob portion flange 70 of the aforementioned second knob portion 64 may be applied to the opening portion of an endoscope with the plug like the forceps opening portion plug body 63, that is, the opening portion that has the conduit one end of which communicates with the injection port into which a liquid is injected, and the other end of which communicates with a discharge port which is different from the injection port and from which the liquid is discharged arranged internally, communicates with a midpoint of the conduit, and is different from the injection port and the liquid discharge port, similarly to the cylinders 31 and 32 in the present embodiment.

The present application is filed claiming the priority of Japanese Patent Application No. 2011-246740 filed in Japan on November 10, 2011, and the above described disclosure is incorporated by reference in the present description, claims and drawings,

## Claims

1. An endoscope plug member installed in an opening portion of an endoscope having the opening portion which communicates with an outside, the endoscope plug member comprising:
an opening portion side contact surface provided at the opening portion;
a plug body having a plug body side contact surface that blocks the opening portion by contacting the opening portion side contact surface;
attaching and holding means that holds the plug body movably in a direction in which the plug body side contact surface moves apart from the opening portion side contact surface, and is attachable to and detachable from the endoscope;
a plug body urging member that gives an urging force that presses the plug body in a direction in which the plug body side contact surface contacts the opening portion side contact surface to the plug body; and
a movable range adjusting portion that selectively changes a movable range of the plug body in the direction to move apart.

2. The endoscope plug member according to claim 1,
wherein the endoscope has an injection port into which a fluid is injected, a discharge port from which the fluid is discharged, and an internal conduit that communicates with the injection port and the discharge port,
the opening portion communicates with the internal conduit partway through the internal conduit, and
the movable range adjusting portion selectively changes an amount of the fluid injected into the internal conduit leaking from between the opening portion and the plug body.

3. The endoscope plug member according to claim 1 or 2,
wherein the movable range adjusting portion is configured by including a cam contact portion provided at the attaching and holding means, a cam portion that is provided at the plug body and has a plurality of cam surfaces having different heights in a movable direction of the plug body, and a knob portion that selectively disposes any one of the plurality of cam surfaces at a position where any one of the plurality of cam surfaces can contact the cam contact portion, and
selectively changes the movable range of the plug body by selectively disposing any one of the plurality of cam surfaces at the position where any one of the plurality of cam surfaces can contact the cam contact portion by the knob portion.

4. The endoscope plug member according to claim 1 or 2,
wherein the movable range adjusting portion is configured by including a flange portion provided at the plug body, a flange contact portion that contacts the flange portion in a movable direction of the plug body and is provided at the attaching and holding means, and a plurality of spacers that are detachably mounted between the flange portion and the flange contact portion, and
selectively changes the movable range of the plug body by mounting none of the spacers between the flange portion and the flange contact portion, or selectively mounting any one of the spacers.

5. The endoscope plug member according to claim 1 or 2,
wherein the movable range adjusting portion is configured by including a plug body contact portion provided at the plug body, and a plug body butting member provided to be capable of being inserted and extracted in a movable range of the plug body contact portion by mobility of the plug body, and
selectively changes the movable range of the plug body by inserting and extracting the plug body butting member in the movable range of the plug body contact portion.

6. The endoscope plug member according to claim 5, further comprising:
a female screw provided at the attaching and holding means, and a male screw that is arranged at the plug body butting member, and is screwed into the female screw in a movable direction of the plug body,
wherein a screwing amount of the male screw into the female screw is selectively changed, whereby the movable range of the plug body is selectively changed.

7. The endoscope plug member according to claim 1 or 2,
wherein the movable range adjusting portion includes a plug body contact portion provided at the plug body, and an additional urging member that is provided to be capable of being inserted and extracted in a movable range of the plug body contact portion by mobility of the plug body, and gives an additional urging force to the plug body in the same direction as an urging force of the plug body urging member, and
selectively changes the movable range of the plug body at a time when pressure given to the plug body by a fluid injected into an internal conduit of the endoscope is a predetermined pressure, by inserting and extracting the additional urging member in the movable range of the plug body contact portion.
